# EUROPEAN PATENT SPECIFICATION

(11) **EP 2 994 080 B1**
(45) Date of publication and mention of the grant of the patent: **07.07.2021**
(21) Application number: 14730672.4
(22) Date of filing: 07.05.2014
(51) Int. Cl.: A61F 6/00, B65D 55/06

(54) **DAMAGE EVIDENT CONDOM PACKAGING**
KONDOMVERPACKUNG MIT SCHADENSEVIDENZ
EMBALLAGE DE PRÉSERVATIF À INDICATEUR D'ENDOMMAGEMENT

(30) Priority: 07.05.2013 US 201361820315 P; 27.03.2014 US 201461971187 P
(43) Date of publication of application: 16.03.2016
(73) Proprietor: Baby Blue Brand Corp., Bala Cynwyd, Pennsylvania 19004 (US)
(72) Inventor: GAINES, L. Kris, Norfolk, Virginia 23518 (US); JACQUES, Veonous M., Philadelphia, Pennsylvania 19119 (US); JACQUES, Auguste, Philadelphia, Pennsylvania 19119 (US); GAINES, David A., Norfolk, Virginia 23518 (US); MORRISON, Crystal G., Pittsburgh, Pennsylvania 15127 (US)
(74) Representative: Perani & Partners S.p.A.
(86) International application number: PCT/US2014/037172
(87) International publication number: WO 2014/182828

(56) References cited:
- EP-A2- 0 629 497
- WO-A1-96/29262
- WO-A1-03/037232
- US-A- 4 516 679
- US-A- 5 839 592
- US-A1- 2011 308 984

## Description

### CROSS-REFERENCE TO RELATED APPLICATIONS

This application claims the benefit of U.S. Provisional Patent Application Serial No. 61/820,315 filed May 7, 2013 and U.S. Provisional Patent Application Serial No. 61/971,187 filed March 27, 2014.

### FIELD OF THE INVENTION

The present invention relates to condom packaging, and more particularly relates to active and intelligent damage evident condom packaging.

### BACKGROUND INFORMATION

Conventional condom packaging provides an expiration date, but no obvious evidence of tampering. Air bubbles have been used in condom packaging as an indication of whether the packaging has been compromised. However, a need exists for an easy and reliable indication that condom packages have been compromised.
WO 03/037232 describes a condom sack for easy determination of internal and external side of the condom having two square three-ply sides, welded on the edge.
US 5,839,592 discloses a plastic closure formed from a thermoplastic composite having an upper or outer layer which is a strong but flexible plastic material, and a lower layer which is softer and comparably easily deformed particularly at elevated application temperatures. In a composite closure, the disk would be formed from this composite laminate.
US 2011/308984 concerns a security pouch made of plastic, which is used to receive and to transport valuables and which has a lower and an upper plastic layer that are joined to each other, forming a closable access opening.
WO 96/29262 relates to a package for condoms comprising an upper and a lower generally flat parts sealed to each other along their edges and defining a cavity for the condom therebetween, wherein the package has the general appearance and dimensions, except for the thickness, of a standardised credit card.
EP0629497 describes a multi-wall film comprising two layers of barrier film material, wherein the layers are sealed one to another to form an enclosure and there is provided within the enclosure a material which is color sensitive to presence or absence of a gas so that, when the film is severed, the gas to which the material is sensitive escapes from or enters in the enclosure and the material thereby changes color and the color change is visible. US 4,516,679 discloses a tamper-proof wrap for encasing a consumer product for shelf display comprising, interior and exterior sheets of a thin plastic material positioned over one another and bonded together around the outer edge and including a layer of a select chemical sealed therebetween such that on puncture of a wrap exterior sheet, a chemical response will occur that will produce a visual indication at the wrap puncture; and distinctive seal means fixed at spaced intervals over the wrap exterior sheet.

### SUMMARY OF THE INVENTION

An aspect of the present invention is to provide a condom package as defined in claim 1.

A further aspect of the present invention is to provide a method of providing an indication that a condom package has been damaged comprising wrapping a condom with a condom package according to claim 1.

Preferred embodiments are defined by the dependent claims.

These and other aspects of the present invention will be more apparent from the following description.

### BRIEF DESCRIPTION OF THE DRAWINGS

Fig. 1 is a partially schematic front view of a sealed condom package with a substantially transparent company logo and universally recognized symbol in accordance with an embodiment of the invention.
Fig. 2 is a partially schematic back view of the sealed condom package of Fig. 1 showing a substantially transparent universal throwaway symbol in accordance with an embodiment of the invention.
Fig. 3 is a partially schematic front view of a condom package once exposed to oxygen with tamper evident coloring displaying a universally recognized symbol as a result of color bloom of a damage indicating material in accordance with an embodiment of the present invention.
Fig. 4 is a partially schematic back view of the condom package of Fig. 3 once exposed to oxygen with tamper evident coloring displaying a universally recognized symbol or customized symbol as a result of the color bloom of the damage indicating material in accordance with an embodiment of the present invention.
Fig. 5 is the flow diagram illustrating a method of making a damage indicating condom package in accordance with an embodiment of the invention.
Fig. 6 is a partially schematic side sectional view showing a portion of a condom packaging material in accordance with an embodiment of the present invention.
Fig. 7 is a partially schematic side sectional view showing a portion of a condom packaging material in accordance with another embodiment of the present invention.

### DETAILED DESCRIPTION

The following is a detailed description of exemplary embodiments to illustrate the principles of the invention. The embodiments are provided to illustrate aspects of the invention, but the invention is not limited to any embodiment. The scope of the invention encompasses numerous alternatives, modifications and equivalents. Numerous specific details are set forth in the following description in order to provide a thorough understanding of the invention. However, the invention may be practiced according to the claims without some or all of these specific details. For the purpose of clarity, technical material that is known in the technical fields related to the invention has not been described in detail so that the invention is not unnecessarily obscured.

The present invention provides active and intelligent condom packaging including information printed thereon that allows anyone seeing it to know that the packaging has been compromised and to throw the product away. A damage indicating material may be utilized that causes the condom packaging to change color when exposed to oxygen, extreme heat and/or excessive pressure such as compression or tension. The packaging gives the user clear indicia that the condom has been compromised and can be immediately discarded. Further features of the invention provide for the information to be printed on a front and/or back layer of the condom packaging. The information may include any one or more of manufacturing information, artwork, text, logos, slogans, insignia, instructions or the like.

The present disclosure also provides a process for manufacturing condom packaging with a film layer of plastic, foil, paper or the like having a damage indicating material applied thereto. The packaging film layer and damage indicating material may be exposed to electromagnetic radiation, which renders the damage indicating material substantially colorless. In certain embodiments, the damage indicating material may be added to a layer of the condom packaging prior to the condom being inserted and the packaging being sealed. A sealing unit may seal the package layers together around each condom and dispense the packaged condoms either individually or in strips. The sealing unit may also print manufacturing information on the sealed packages.

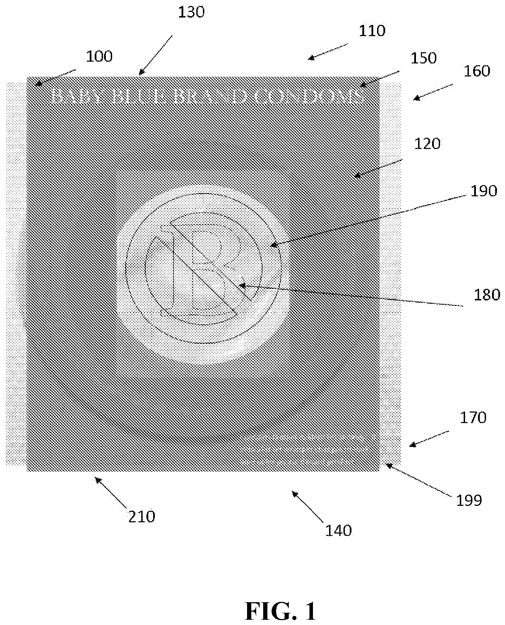

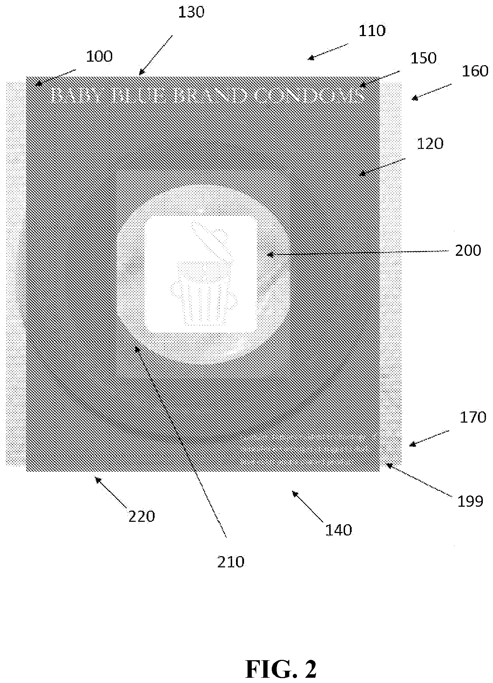

Referring now to the invention in more detail, in Figs. 1 and 2, the combination of a condom and a package therefor is schematically shown at 100. The combination comprises a package shown generally at 110, and a rolled condom shown generally at 120. The package comprises a front wrapper panel or layer 130 and a back wrapper panel or layer 140. The front 130 and back 140 wrapper layers are sealed to one another around their respective edges. Labeling is shown generally at 150. Package instructions are shown generally at 199. In the embodiment shown, the instructions 199 state "Contains tamper evident technology. If indicator on wrapper changes to dark blue color please discard product". As shown in Fig. 1, a front outer layer 210 covers the front wrapper layer 130. A damage indicating material is applied on the front outer layer 210. As shown in Fig. 2, a back outer layer 220 covers the back wrapper layer 140, and a damage indicating material is applied on the back outer layer 220.

Still referring to Figs. 1 and 2, universal symbols 190, 200 and a company logo 180 are printed on the package with a damage indicating material such as methylene blue, alternative oxygen sensitive reactive dye, or alternative damage indicating material on package interiors, and processed to render them transparent. When the reactive damage indicating material is subsequently activated by exposure to oxygen, extreme heat, or excessive pressure, such as compression or tension, it changes in appearance. For example, the reactive damage indicating material changes color when exposed to oxygen. The color change should be sufficiently stable such that the color is visible for a sufficiently long time period. In certain embodiments, the long-term stability may be for periods of days, weeks, months or years.

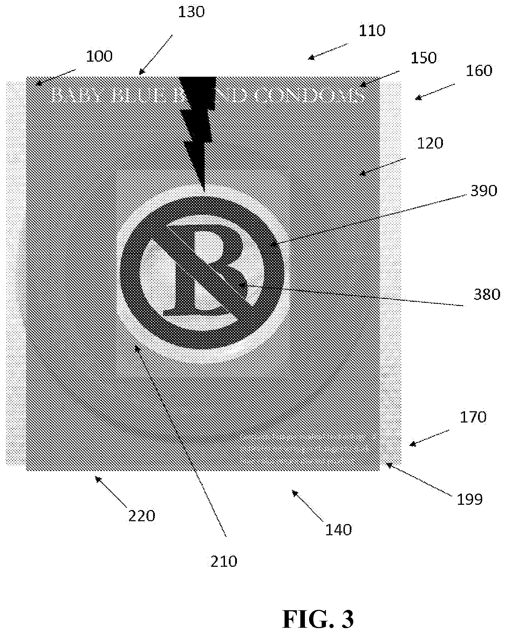

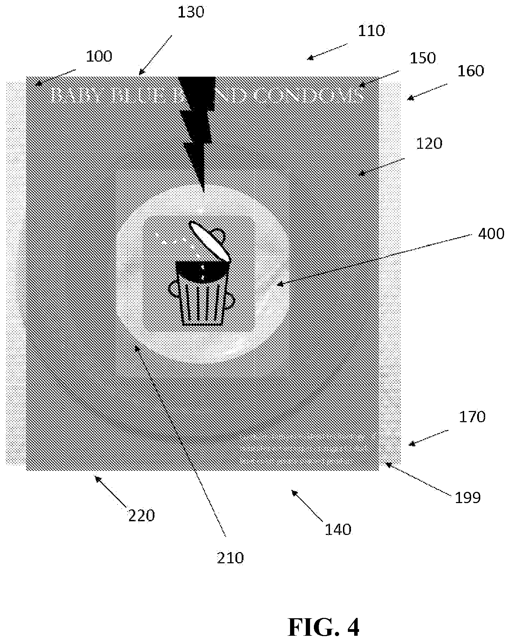

Figs. 3 and 4 schematically illustrate the condom package 100 in a damage-indicating state in which the condom wrapper has been torn. The universal symbols 390, 400 and company logo 380 are re-oxidized due to exposure to oxygen, or bloom with color due to extreme heat, excessive pressure, compression or tension. Re-oxidation is indicated with a color bloom of the universal symbols 390, 400 and company logo 380.

It will be understood that the rolled condom 120 as shown in Figs. 1-4 has an open end 160 and a closed end 170. However, the precise details of the condom 120 are not relevant to the present invention, e.g., the condom may be pre-lubricated or not, may have a tip for collection of ejaculate, etc.

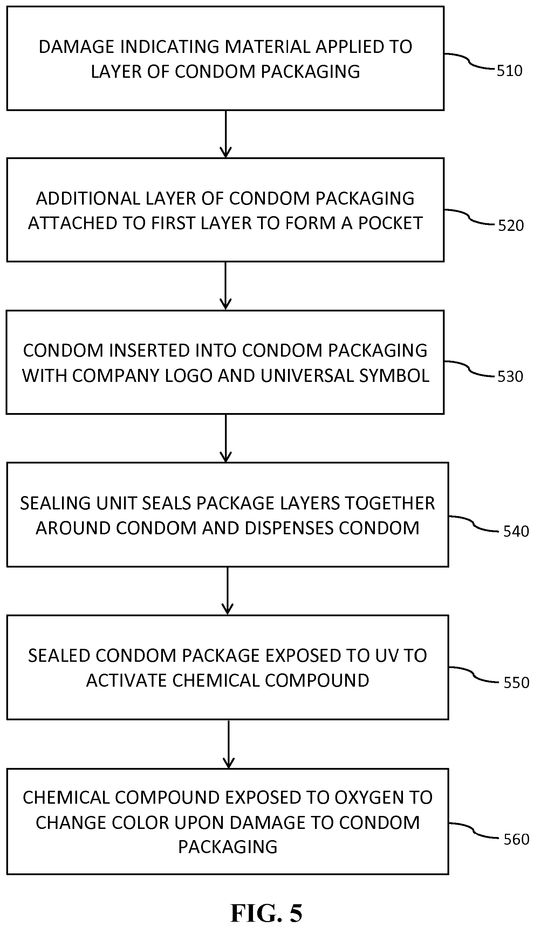

Fig. 5 is a flow diagram illustrating a method of making a damage evident condom package in accordance with an embodiment of the present invention. In step 510, the interior wall of a condom packaging layer is coated with methylene blue or any suitable alternative oxygen-sensitive reactive dyes or damage indicating material. The color-changing material may be applied by any suitable means such as spraying, brushing, screen printing, ink jet printing or the like. Methylene blue dye may be used in the printing process. Alternative dyes may be thionine (Th, thiazine), azure B (AzB, thiazine), nile blue (NR, oxazine), non-toxic phosphorous pigmentation, or any alternative oxygen sensitive reactive dyes or any dyes that change color when exposed to oxygen, extreme heat, or excessive pressure, compression or tension. The damage indicating material may comprise more than one type of reactive dye. There will be associative processes to prepare the oxygen sensitive reactive dyes to reduce it to a colorless form. The printer may be of conventional construction and operation and sprays dye onto the packaging. The manufacturing information is provided by a processor (not shown), the operation of which is not germane to this invention, and which also controls printing by the sealing unit.

In step 520, another layer, which may also have damage indicating material applied thereto, is sealed or otherwise attached to the layer formed in step 510, e.g., to thereby form a pocket.

In step 530, a condom is inserted into the condom package or pocket. The condom may optionally be pre-wrapped in any suitable type of wrapper prior to insertion into the pocket. A company logo and/or universal symbols may be printed on one or more of the layers of packaging.

In step 540, a sealing unit seals the package layers together around each condom 120 and dispenses the condoms either individually or in strips.

In step 550, the sealed condom package is exposed to electromagnetic radiation, such as ultraviolet radiation or any other radiation of suitable wavelength, e.g., to render the damage indicating material substantially colorless.

In step 560, the chemical compound is exposed to oxygen to change the color of the damage indicating material by oxidation or another type of chemical reaction that changes the reactive dye from colorless to colored upon tampering, extreme heat, excessive pressure, compression, tension or any other breach of the condom wrapper such as a tear, pin-prick, or intentionally opening condom packaging. Printing between the layers of packaging prevents the dye from coming into contact with the condom. Is thus not possible for a user to experience any reaction due to contact with the dye.

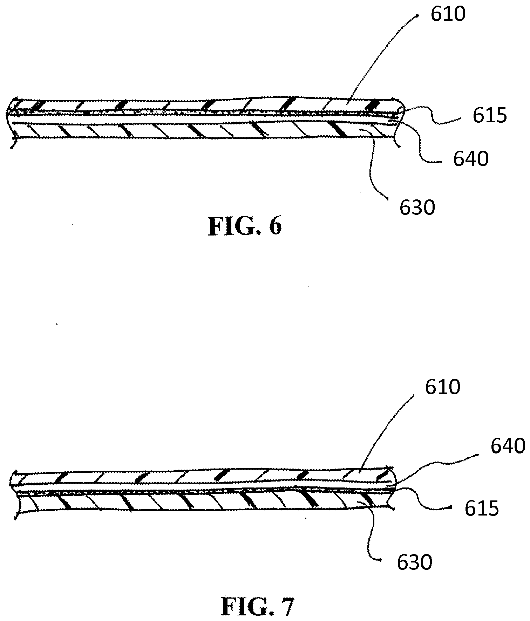

Figs. 6 and 7 are partially schematic side sectional views illustrating various condom packaging layers in accordance with embodiments of the present invention. In Fig. 6, an outer layer 610 is provided with a layer of damage indicating material 615 applied thereto. An inner condom wrapper layer 630 is located adjacent to the outer layer 610 and damage indicating material 615. In certain embodiments, the inner wrapper layer 630 is not adhered to the layer of damage indicating material 615, as shown by the gap 640 in Fig. 6. Although the gap 640 is shown as a physical spacing between the layers 630 and 615 in Fig. 6, it should be recognized that the gap may be closed such that the layers 630 and 615 contact each other. For example, when the space between the outer layer 610 and inner wrapper layer 630 is evacuated, the layer of damage indicating material 615 would typically contact the underlying inner wrapper layer 630. Alternatively, when the space between the outer layer 610 and inner wrapper layer 630 is filled with an inert or non-reactive gas, the pressure of the gas may result in the formation of a physical gap 640, as shown in Fig. 6.

The embodiment shown in Fig. 7 is similar to the embodiment of Fig. 6, except the layer of damage indicating material 615 is applied to the outer surface of the inner wrapper layer 630 rather than the inner surface of the outer layer 610.

In accordance with embodiments of the present invention, the various inner condom wrapper layers and outer layers may be made of any suitable materials such as polymeric films, foils, paper and the like. Some examples of polymeric layers include cellulosic materials, vinyl polymers such as methyacrylates, polyolefins such as polyethylene, polyethylene terephthalate (PET), ethylene vinyl acetate copolymers, polyethylene, nylon (polyamide) and the like. The inner wrapper layers and outer layers may be made of the same or different materials. In certain embodiments, the inner wrapper layers may comprise foil coated with any of the aforementioned polymers, or such polymers alone. In certain embodiments, the outer layers may comprise polyethylene or the like, which may optionally be coextruded with nylon or the like.

In an embodiment of the invention, a condom is disposed in an inner wrapper, an outer wrapper surrounds the inner wrapper, and a layer of color-changing material is applied to the inner surface of the outer wrapper. Alternatively, as described above, the outer surface of the inner wrapper may have the color-changing layer of material applied thereto. In both of these embodiments, the space between the inner and outer wrappers may be evacuated by any suitable type of vacuum source in order to remove gasses including oxygen from the space between the wrappers. In this embodiment, when the outer wrapper is punctured, torn or otherwise breached, air will fill the previously evacuated space between the inner and outer wrappers, thereby coming into contact with the relatively large surface areas of the wrappers, i.e., the outer surface of the inner wrapper will be exposed to air and the inner surface of the outer wrapper will be exposed to air. The presence of the color-changing material on the inner surface of the outer wrapper and/or on the outer surface of the inner wrapper will thereby provide an indication that the outer wrapper has been punctured, torn or otherwise breached and that air has entered the space between the wrappers. As an alternative to evacuating the space between the inner and outer wrappers, the inner space may be at least partially filled with an inert or non-reactive gas such as nitrogen or the like that does not cause the color-changing material to react and change colors.

In accordance with an embodiment of the present invention, dual-wrapper arrangements as described above may be made by providing a pre-packaged condom in the inner wrapper, followed by applying the outer wrapper around the inner wrapper. For example, the outer wrapper may be provided as a pre-formed pocket in which the inner wrapper containing the condom is inserted, followed by sealing of the open end of the outer wrapper. As discussed above, before, during or after the sealing operation, the space between the inner and outer wrappers may be evacuated and/or filled with a non-reactive gas. As another example, separate sheets of outer wrapper material may be placed on opposite sides of the inner wrapper containing the condom, following by sealing of the peripheral edges of the outer wrapper layers together to thereby seal the inner wrapper and condom within the outer wrapper. Again, the space between the inner and outer wrappers may be evacuated and/or filled with a non-reactive gas during the sealing operation. Such operations, in which the condom is first sealed in the inner wrapper followed by sealing an outer wrapper around the inner wrapper, may be conducted contemporaneously with each other, e.g., the inner and outer wrappers may be applied in the same manufacturing operation. Alternatively, pre-packaged condoms may be modified by applying the outer wrapper at a different time or location, e.g., at a different facility from the original condom manufacturing location.

In accordance with another embodiment of the present invention, a single condom wrapper is provided with multiple laminated layers in which at least one of the layers contains the color-changing material. For example, a layer of color-changing material may be sandwiched between inner and outer polymeric layers to provide a composite wrapper structure with color-changing capabilities. As another example, a layer of color-changing material may be applied on the inner surface of the single condom wrapper. In this embodiment, the color changing material layer would be exposed to the condom, and the color-changing material must be non-reactive with the material of the condom or any other liquids or gasses contained within the wrapper, and the color-changing layer must not damage the condom or vice versa.

The damage indicating material may be printed or applied by any suitable means such as spraying, screen printing, brushing, immersion, ink jet printing, or the like. The damage indicating material may comprise methylene blue, alternative oxygen-sensitive reactive dyes, or any alternative dyes that are substantially colorless in the absence of oxygen. Alternative dyes may be thionine (Th, thiazine), azure B (AzB, thiazine), nile blue (NR, oxazine), non-toxic phosphorous pigmentation, alternative oxygen sensitive reactive dyes, or any dyes that change color when exposed to oxygen, extreme heat, or excessive pressure, compression or tension.

The packaging in its completed form when taken directly out of the box may have a company logo displayed clear on one side and on the reverse a clear icon of a trashcan. If opened for intended use or accidentally or intentionally pricked exposing the condom inside, exposed to extreme heat, or excessive compression or tension, the coloring of the packaging will change, highlighting the company logo with universal "NO" symbols emblazoned over it, including, but not limited to the symbols for "Do not Enter", "Prohibited", an "X", a circle, square, triangle with a backslash or line going through it, horizontally, vertically, or diagonally, a stop sign, a hand, trash can or customized symbol. On the reverse side a trash can icon or alternative customized symbol(s) will also color change indicating that the packaging and the condom contained within should be thrown away.

The disclosed embodiments are illustrative, not restrictive. While specific configurations of the condom have been described, it is understood that the present invention can be applied to a wide variety of condom packaging to include paper, foils, or plastics, as well as any combination thereof, such as foil-lined paper, plastic-lined paper or a wax-lined paper. The package may take a variety of forms such as rectangular, oval, etc., or can be male or female condom packaging. The package may be provided with a separation structure, such as an edge tear area, a zipper-locked edge area, or an openable, adhesively sealed edge area. There are many alternative ways of implementing the invention. Alternative embodiments include transparent or translucent plastic lids, transparent or translucent plastic containers. Plastics can be LDPE, HDPE, PP, or a combination of plastics including, but not limited to polycarbonates, or acrylics.

The following example is intended to illustrate various aspects of the present invention, and is not intended to limit the scope of the invention.

### Example

A color-changing material was made as follows. Ten grams of 5% aqueous solution of hydroxethylcellulose (HEC) and 2.5 grams of 5% aqueous dispersion of titanium dioxide (TiO₂) were added to a 20 mL amber glass scintillation vial. The mixture was sonicated for approximately 30 minutes in a warm ultrasonic bath. After dispersing the TiO₂, 0.5 grams of 5% aqueous solution of methylene blue (MB) was added to the mixture. The mixture was sonicated or magnetically stirred for 10-15 minutes to disperse the MB in the aqueous mixture. After dispersing the MB into the mixture, 0.15 g of triethanolamine (TEOA) were added using a plastic or glass pipet. The final formulation was sonicated for approximately 30 minutes in a warm ultrasonic bath prior to substrate application.

The formulation was applied as a thin layer of glass or plastic film substrate and was allowed to dry. Standard laboratory glass microscope slides and 3 mil thick co-extruded polyethylene-nylon vacuum packaging film were used as substrates for coating. The plastic film was also used as an overwrap to seal the coated glass slides or plastic film prior to activation and deactivation. The formulation was typically applied by painting a thin film on the substrate with a paint brush. Uniform film casting can also be accomplished using a spin coater or K bar techniques. The coated substrates were allowed to dry within a dark oven set at 50°C for 16 hours. The resultant blue layer had a dry-film thickness of approximately 2 mils or less with variations in thickness dependent on the exact formulation. The dried, blue films of color-changing material were vacuum sealed within a plastic film overwrap. Following evacuation and thermal sealing, the material was "activated" under a UV lamp to convert the blue MB form to a white leuco MB form. Upon such UV exposure, the layer changed from substantially blue to off-white or light gray. After activation, the layer was exposed to air by puncturing or cutting open the vacuum overwrap, resulting in a change back to the blue color. After the color transformation, the layer retained a significant degree of its blue color for over several weeks.

Whereas particular embodiments of this invention have been described above for purposes of illustration, it will be evident to those skilled in the art that numerous variations of the details of the present invention may be made without departing from the invention as defined in the appended claims.

## Claims

1. A condom package (110) comprising:
an inner wrapper layer (630) defining an interior volume structured and arranged to receive a condom (120); and
an outer layer (610) at least partially covering the inner wrapper layer (630);
**characterized in that** a gap (640) is provided between the inner wrapper layer (630) and the outer layer (610); and
a damage indicating material (615) is provided between the inner wrapper layer (630) and the outer layer (610) and adjacent to the gap (640).

2. The condom package (110) of claim 1, wherein the damage indicating material (615) changes color when exposed to oxygen.

3. The condom package (110) of claim 1, wherein the damage indicating material (615) comprises methylene blue.

4. The condom package (110) of claim 1, wherein the damage indicating material (615) is applied to the outer layer (610).

5. The condom package (110) of Claim 1, wherein the damage indicating material (615) is applied to an interior surface of the outer layer (610).

6. The condom package (110) of claim 1, wherein the damage indicating material (615) is applied to the inner wrapper layer (630).

7. The condom package (110) of claim 6, wherein the damage indicating material (615) is applied to an exterior surface of the inner wrapper layer (630).

8. The condom package (110) of claim 1, wherein the outer layer (610) is coextensive with the inner wrapper layer (630).

9. The condom package (110) of claim 1, wherein the gap (640) is closed such that the damage indicating material (615) contacts the inner wrapper layer (630) and the outer layer (610).

10. The condom package (110) of claim 9, wherein the gap (640) between the inner wrapper layer (630) and the outer layer (610) is evacuated.

11. The condom package (110) of claim 1, wherein the gap (640) between the inner wrapper layer (630) and the outer layer (610) is at least partially filled with an inert gas.

12. The condom package (110) of claim 1, wherein the damage indicating material (615) is applied to the inner wrapper layer (630) or the outer layer (610) in the form of indicia (180, 190, 200, 380, 390, 400).

13. The condom package (110) of claim 12, wherein the indicia includes a warning symbol (190, 390).

14. The condom package (110) of claim 12, wherein the indicia includes instructions to discard the package (200, 400).

15. A method of providing an indication that a condom package (100) has been damaged comprising wrapping a condom (120) with a condom package (110) according to claim 1.

## Patentansprüche

1. Eine Kondomverpackung (110) umfassend:
eine innere Hüllschicht (630), die ein Innenvolumen definiert, das strukturiert und angeordnet ist, um ein Kondom (120) aufzunehmen; und
eine äußere Schicht (610), die die innere Hüllschicht (630) zumindest teilweise bedeckt;
**dadurch gekennzeichnet ist, dass** zwischen der inneren Hüllschicht (630) und der äußeren Schicht (610) ein Spalt (640) vorgesehen ist; und dass
zwischen der inneren Hüllschicht (630) und der äußeren Schicht (610), sowie benachbart zu dem Spalt (640), ein beschädigungsanzeigendes Material (615) vorgesehen ist.

2. Kondomverpackung (110) nach Anspruch 1, wobei das beschädigungsanzeigende Material (615) die Farbe ändert, wenn es Sauerstoff ausgesetzt wird.

3. Kondomverpackung (110) nach Anspruch 1, wobei das beschädigungsanzeigende Material (615) Methylenblau enthält.

4. Kondomverpackung (110) nach Anspruch 1, wobei das beschädigungsanzeigende Material (615) auf die äußere Schicht (610) aufgebracht wird.

5. Kondomverpackung (110) nach Anspruch 1, wobei das beschädigungsanzeigende Material (615) auf eine Innenfläche der äußeren Schicht (610) aufgebracht wird.

6. Kondomverpackung (110) nach Anspruch 1, wobei das beschädigungsanzeigende Material (615) auf die innere Hüllschicht (630) aufgebracht wird.

7. Kondomverpackung (110) nach Anspruch 6, wobei das beschädigungsanzeigende Material (615) auf eine Außenfläche der inneren Hüllschicht (630) aufgebracht wird.

8. Kondomverpackung (110) nach Anspruch 1, wobei die äußere Schicht (610) mit der inneren Hüllschicht (630) koextensiv ist.

9. Kondomverpackung (110) nach Anspruch 1, wobei der Spalt (640) so geschlossen ist, dass das beschädigungsanzeigende Material (615) die innere Hüllschicht (630) und die äußere Schicht (610) berührt.

10. Kondomverpackung (110) nach Anspruch 9, wobei der Spalt (640) zwischen der inneren Hüllschicht (630) und der äußeren Schicht (610) evakuiert wird.

11. Kondomverpackung (110) nach Anspruch 1, wobei der Spalt (640) zwischen der inneren Hüllschicht (630) und der äußeren Schicht (610) zumindest teilweise mit einem Inertgas gefüllt wird.

12. Kondomverpackung (110) nach Anspruch 1, wobei das beschädigungsanzeigende Material (615) in Form von Angaben (180, 190,200, 380,390,400) auf die innere Hüllschicht (630) oder die äußere Schicht (610) aufgebracht wird.

13. Kondomverpackung (110) nach Anspruch 12, wobei die Angaben ein Warnsymbol (190, 390) beinhalten.

14. Kondomverpackung (110) nach Anspruch 12, wobei die Angaben Anweisungen zum Entsorgen der Packung (200, 400) beinhalten.

15. Verfahren zum Bereitstellen eines Hinweises, dass eine Kondomverpackung (100) beschädigt wurde, umfassend das Umhüllen eines Kondoms (120) mit einer Kondomverpackung (110) nach Anspruch 1.

## Revendications

1. Emballage de préservatif (110) comprenant :
une couche d'enveloppe intérieure (630) définissant un volume intérieur structuré et disposé pour recevoir un préservatif (120) ; et
une couche extérieure (610) qui recouvre au moins partiellement la couche d'enveloppe intérieure (630) ;
**caractérisé en ce qu'**un espace vide (640) est fourni entre la couche d'enveloppe intérieure (630) et la couche extérieure (610) ; et
un matériau indicateur d'endommagement (615) est fourni entre la couche d'enveloppe intérieure (630) et la couche extérieure (610) et adjacent à l'espace vide (640).

2. Emballage de préservatif (110) de la revendication 1, dans lequel le matériau indicateur d'endommagement (615) change de couleur lorsqu'il est exposé à l'oxygène.

3. Emballage de préservatif (110) de la revendication 1, dans lequel le matériau indicateur d'endommagement (615) comprend du bleu de méthylène.

4. Emballage de préservatif (110) de la revendication 1, dans lequel le matériau indicateur d'endommagement (615) est appliqué à la couche extérieure (610).

5. Emballage de préservatif (110) de la revendication 1, dans lequel le matériau indicateur d'endommagement (615) est appliqué à une surface intérieure de la couche extérieure (610).

6. Emballage de préservatif (110) de la revendication 1, dans lequel le matériau indicateur d'endommagement (615) est appliqué à la couche d'enveloppe intérieure (630).

7. Emballage de préservatif (110) de la revendication 6, dans lequel le matériau indicateur d'endommagement (615) est appliqué à une surface extérieure de la couche d'enveloppe intérieure (630).

8. Emballage de préservatif (110) de la revendication 1, dans lequel la couche extérieure (610) est coextensive à la couche d'enveloppe intérieure (630).

9. Emballage de préservatif (110) de la revendication 1, dans lequel l'espace vide (640) est refermé de telle sorte que le matériau indicateur d'endommagement (615) entre en contact avec la couche d'enveloppe intérieure (630) et la couche extérieure (610).

10. Emballage de préservatif (110) de la revendication 9, dans lequel l'espace vide (640) entre la couche d'enveloppe intérieure (630) et la couche extérieure (610) est évacué.

11. Emballage de préservatif (110) de la revendication 1, dans lequel l'espace vide (640) entre la couche d'enveloppe intérieure (630) et la couche extérieure (610) est au moins partiellement rempli avec un gaz inerte.

12. Emballage de préservatif (110) de la revendication 1, dans lequel le matériau indicateur d'endommagement (615) est appliqué à la couche d'enveloppe intérieure (630) ou la couche extérieure (610) sous forme d'indices (180, 190,200, 380,390,400).

13. Emballage de préservatif (110) de la revendication 12, dans lequel les indices incluent un symbole d'avertissement (190, 390).

14. Emballage de préservatif (110) de la revendication 12, dans lequel les indices incluent des instructions pour jeter l'emballage (200, 400).

15. Procédé de fourniture d'une indication qu'un emballage de préservatif (100) a été endommagé comprenant envelopper un préservatif (120) avec un emballage (110) selon la revendication 1.
